(19) Europäisches Patentamt
European Patent Office
Office européen des brevets

(11) Veröffentlichungsnummer: **0 081 125**
**B1**

(12) # EUROPÄISCHE PATENTSCHRIFT

(45) Veröffentlichungstag der Patentschrift: **08.05.85**

(51) Int. Cl.⁴: **C 09 B 57/06,** C 07 D 209/92, D 06 P 1/42, D 21 H 3/80, D 01 F 1/06

(21) Anmeldenummer: **82110730.7**

(22) Anmeldetag: **20.11.82**

(54) **Kationische Farbstoffe, Verfahren zu ihrer Herstellung sowie ihre Verwendung zum Färben und Massefärben.**

(30) Priorität: **04.12.81 DE 3148104**

(43) Veröffentlichungstag der Anmeldung: **15.06.83 Patentblatt 83/24**

(45) Bekanntmachung des Hinweises auf die Patenterteilung: **08.05.85 Patentblatt 85/19**

(84) Benannte Vertragsstaaten: **CH DE FR GB LI**

(56) Entgegenhaltungen:
**DE - A - 2 921 690**
**DE - B - 2 366 174**
**FR - A - 2 247 513**

(73) Patentinhaber: **BAYER AG, Konzernverwaltung RP Patentabteilung, D-5090 Leverkusen 1 Bayerwerk (DE)**

(72) Erfinder: **Kühlthau, Hans-Peter, Dr., Paul-Klee-Strasse 48, D-5090 Leverkusen 1 (DE)**
Erfinder: **Harnisch, Horst, Dr., Heinenbusch 4, D-5203 Much (DE)**

EP 0 081 125 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Gegenstand der vorliegenden Erfindung sind kationische Farbstoffe der allgemeinen Formel

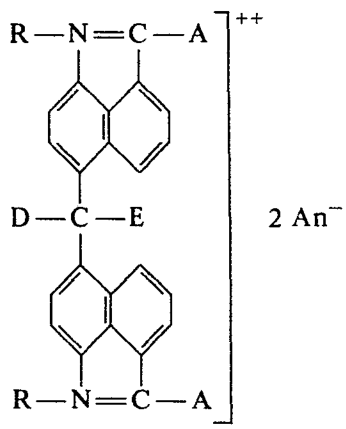

(I)

sowie ihre Basen

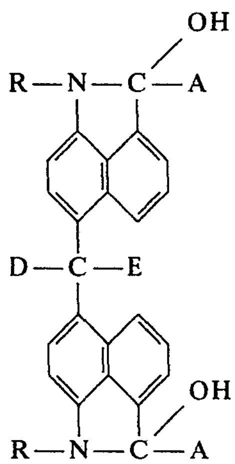

(Ia) bzw.

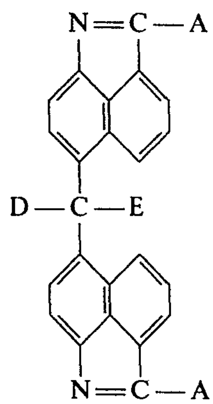

(Ib)

worin

R für Wasserstoff oder einen gegebenenfalls durch nichtionische Reste substituierten $C_1$- bis $C_8$-Alkylrest,

A für einen Rest der Formeln

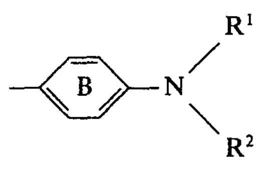

(II) oder

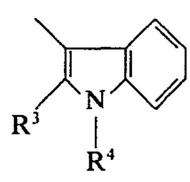

(III)

worin

$R^1$ Wasserstoff, einen gegebenenfalls durch nichtionische Reste substituierten $C_1$- bis $C_6$-Alkylrest oder Cycloalkyl und

$R^2$ einen gegebenenfalls durch nichtionische Reste substituierten $C_1$- bis $C_6$-Alkylrest, der gegebenenfalls einen Ring zum Ring B schließen kann, oder einen Aralkyl- oder Arylrest darstellen, oder

$R^1$ und $R^2$ zusammen mit dem N-Atom einen Ring bilden,

$R^3$ einen $C_1$- bis $C_4$-Alkylrest, einen $C_1$- bis $C_4$-Alkoxycarbonylrest oder einen Arylrest,

$R^4$ Wasserstoff oder einen gegebenenfalls durch nichtionische Reste substituierten $C_1$- bis $C_4$-Alkylrest bedeuten,

D und E für Wasserstoff, einen gegebenenfalls durch nichtionische Reste substituierten $C_1$- bis $C_4$-Alkyl- oder $C_2$- bis $C_3$-Alkenylrest und D zusätzlich für Aryl, Aralkyl, Aralkenyl oder einen heterocyclischen Rest stehen, oder D und E zusammen mit dem C-Atom einen Ring bilden, und

An für ein Anion steht,

und worin die Ringe in der Farbstoffchemie übliche nichtionische Reste enthalten können, sowie ein Verfahren zur Herstellung dieser Farbstoffe und ihre Verwendung zum Färben von Papiermassen, von kationisch färbbaren Fasern aus Polymerisaten und Mischpolymerisaten sowie von sauer modifizierten Fasern und Polyestern.

Als nichtionische Substituenten kommen im Rahmen dieser Erfindung besonders Halogenatome, Nitril-, Carbonsäureester- und -amidgruppen, Hydroxy-, Acyloxy-, Alkoxy-, Aralkoxy-, Aryloxygruppen und die analogen Mercaptogruppen, Aminogruppen und durch Alkyl-, Aralkyl-, Aryl-, Cycloalkyl- oder Acylgruppen mono- oder disubstituierte Aminogruppen, Acylreste, Sulfonamidgruppen und — an Ringen — Nitro- und Alkylgruppen in Betracht, aber auch Carboxylgruppen, die im neutralen und sauren pH-Bereich sehr wenig dissoziieren und den kationischen Charakter der Farbstoffe deshalb nicht beeinflussen.

Im Rahmen der Erfindung wird — soweit nicht gesondert angegeben — unter einem Alkylrest bevorzugt ein Rest mit 1—4 C-Atomen verstanden.

Insbesondere stehen Aryl für Phenyl und Aralkyl für Benzyl oder Phenylethyl, die ihrerseits durch die genannten nichtionischen Reste substituiert sein können.

Bevorzugtes Cycloalkyl sind Cyclopentyl und Cyclohexyl, die durch $C_1$- bis $C_4$-Alkyl substituiert sein können.

Unter Acyl wird beispielsweise Acetyl, Propionyl, Benzoyl, $C_1$- bis $C_4$-Alkylsulfonyl und Phenylsulfonyl verstanden.

Halogen steht vorzugsweise für Chlor und Brom.

Geeignete heterocyclische Ringe D gehören beispielsweise der Furan-, Thiophen-, Indol-, Pyridin-, Pyrimidin- oder Chromenon-Reihe an.

Hervorzuheben sind Farbstoffe der Formel

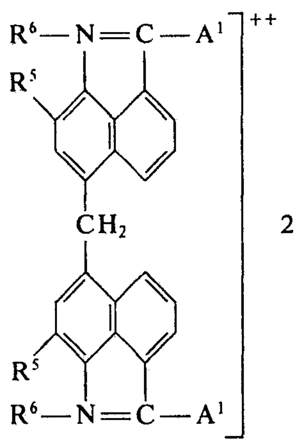

2 $An^-$ (IV)

worin

$R^6$ für Wasserstoff, $C_1$- bis $C_4$-Alkyl, das durch Cyan, Hydroxy, $C_1$- bis $C_4$-Alkoxy, Chlor, $C_1$- bis $C_4$-Alkoxycarbonyl, Aminocarbonyl oder Mono- oder Di-$C_1$- bis $C_4$-alkylaminocarbonyl substituiert sein kann,

$R^5$ für Wasserstoff, Chlor oder Brom,

$A^1$ für

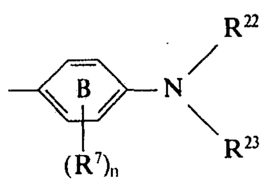

(V)

worin

$R^{22}$ Wasserstoff oder einen gegebenenfalls durch 1—3 Hydroxy, 1—3 Halogen, $C_1$- bis $C_4$-Alkoxy, Cyan, $C_1$- bis $C_4$-Alkylcarbonyloxy, Hydroxy-$C_1$- bis $C_4$-alkoxy, Aminocarbonyl, Carboxy, $C_1$- bis $C_4$-Alkoxycarbonyl oder $C_3$- oder $C_4$-Alkenyloxy,

durch Acyloxy, Benzyloxy, Phenyloxy, Sulfonamido oder Acylamido substituierten $C_1$- bis $C_6$-Alkylrest, gegebenenfalls durch Halogen substituierten $C_2$- bis $C_4$-Alkenylrest oder gegebenenfalls durch $C_1$- bis $C_4$-Alkylrest substituierten Cyclohexylrest,

$R^{23}$ einen gegebenenfalls wie $R^{22}$ substituierten $C_1$- bis $C_6$-Alkylrest, Benzyl oder Phenyl, oder zusammen mit dem Ring B ein Tetrahydrochinolin, Indolin, Hexahydrocarbazol oder Dihydrobenzoxazin, die $C_1$- bis $C_4$-Alkyl- und Phenylgruppen tragen können, oder

$R^{22}$ und $R^{23}$ zusammen einen Pyrrolidin-, Piperidin-, Morpholin-, Pyrazolin- oder Piperazinring, die $C_1$- bis $C_4$-Alkyl- und Phenylgruppen tragen können,

$R^7$ $C_1$- bis $C_4$-Alkyl, $C_1$- bis $C_4$-Alkoxy oder Halogen und

n 1 bis 4 bedeuten,

oder für

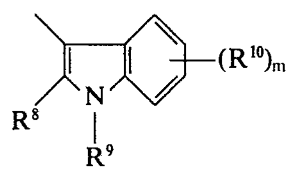

(VI)

worin

$R^8$ einen Alkylrest mit 1 bis 4 C-Atomen, einen Phenylrest oder einen Alkoxycarbonylrest mit 1 bis 4 C-Atomen,

$R^9$ Wasserstoff, einen gegebenenfalls durch Hydroxy, Halogen, Alkoxy mit 1 bis 4 C-Atomen, Cyan oder Acyloxy substituierten Alkylrest mit 1 bis 4 C-Atomen und

$R^{10}$ Wasserstoff, Halogen, $C_1$- bis $C_4$-Alkyl, $C_1$- bis $C_4$-Alkoxy, Carbalkoxy mit 1 bis 4 C-Atomen, $C_1$- bis $C_4$-Alkylsulfonyl, Phenylsulfonyl, Acetyl oder Benzoyl und

m 1 bis 4 bedeuten, und

$An^-$ für ein Anion stehen, und worin die Phenylreste durch $C_1$- bis $C_4$-Alkyl, $C_1$- bis $C_4$-Alkoxy, Hydroxy, Chlor oder Brom substituiert sein können.

Von den Farbstoffen der Formel IV sind diejenigen bevorzugt, worin

$R^6$ für Wasserstoff, $C_1$- bis $C_4$-Alkyl, $\beta$-Cyanethyl, $\beta$-Hydroxyethyl, $\beta$-Alkoxyethyl, $\beta$-Chlorethyl, Mehoxycarbonylmethyl oder Ethoxycarbonylmethyl,

$R^5$ für Wasserstoff und

$A^1$ für

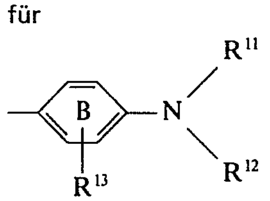

(VII)

worin

$R^{11}$ Wasserstoff oder einen gegebenenfalls durch Hydroxy, Methoxy, Ethoxy, Chlor, Cyan, Acetoxy oder Acetyl substituierten $C_1$- bis $C_4$-Alkylrest,

$R^{12}$ einen gegebenenfalls durch Hydroxy, Methoxy, Ethoxy, Chlor, Cyan, Acetoxy oder Acetyl substituierten $C_1$- bis $C_4$-Alkylrest, Benzyl oder gegebenenfalls durch Chlor, Methyl, Methoxy oder Ethoxy substituiertes Phenyl,

$R^{13}$ Wasserstoff, Methyl, Chlor, Methoxy oder Ethoxy bedeuten,

für

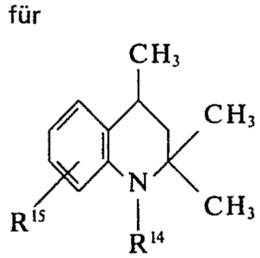

(VIII)

worin

$R^{14}$ Wasserstoff oder $C_1$- bis $C_4$-Alkyl, $C_2$- bis $C_4$-Hydroxyalkyl, Chlorethyl, Bromethyl, Methoxyethyl, Cyanethyl, Acetoxyethyl, Hydroxyethoxyethyl, Aminocarbonylethyl, Carboxyethyl, γ-Cyanpropyl, β-Hydroxy-γ-allyloxy-n-propyl, β-Hydroxy-γ-methoxy-n-propyl, β-Hydroxy-γ-ethoxy-n-propyl, β-Hydroxy-γ-butoxy-n-propyl, β-Chlor-n-propyl, β-Chlor-n-butyl, β-Chlori-butyl, β, γ-Dichlor-n-propyl, β-Acetoxy-n-propyl, β-Hydroxy-γ-chlor-n-propyl, Vinyl, Allyl, Methallyl, Chlorallyl oder Cyclohexyl und
$R^{15}$ Wasserstoff, Methoxy oder Ethoxy bedeuten,

oder für

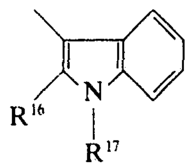

(IX)

worin

$R^{16}$ Methyl oder gegebenenfalls durch Chlor, Methyl oder Methoxy substituiertes Phenyl und
$R^{17}$ einen gegebenenfalls durch Hydroxy, Chlor, Cyan oder Acyloxy substituierten $C_1$- bis $C_4$-Alkylrest oder Wasserstoff bedeuten,

stehen.

Insbesondere bevorzugt sind die Farbstoffe der allgemeinen Formel

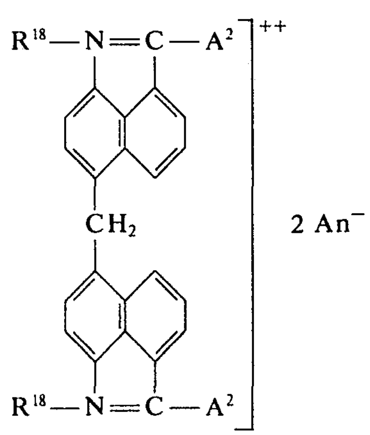

(X)

worin

$R^{18}$ für Methyl, Ethyl oder β-Cyanethyl und
$A^2$ für

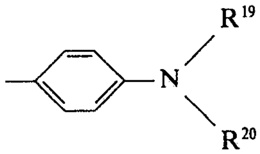

(XI)

worin

$R^{19}$ Wasserstoff, Methyl, Ethyl, Cyanethyl, Hydroxyethyl, Acetoxyethyl, Methoxyethyl, Ethoxyethyl, Chlorethyl, Hydroxypropyl, Propyl oder Butyl und
$R^{20}$ eine der für $R^{19}$ genannten Alkylgruppen oder Phenyl, Methoxyphenyl, Ethoxyphenyl, Methylphenyl oder Chlorphenyl bedeuten,

oder ganz besonders bevorzugt für

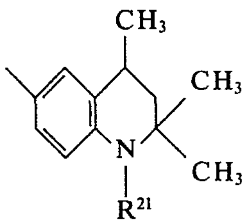

(XII)

worin

$R^{21}$ Wasserstoff, Methyl, Ethyl, Propyl, Butyl, Hydroxyethyl, Hydroxypropyl, Cyanethyl, Chlorethyl, Acetoxyethyl, Acetoxypropyl, Methoxyethyl, Ethoxyethyl, Hydroxybutyl, Hydroxycarbonylethyl oder Hydroxyethoxyethyl bedeutet, und

$An^-$ für ein Anion stehen.

Die neuen Farbstoffe werden hergestellt durch Kondensation eines Naphtholactamderivates der Formel

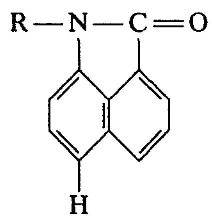

(XIII)

worin

R die oben angegebene Bedeutung hat,

und die Naphtholactamringe wie in Formel (I) angegeben substituiert sein können,

mit einer Verbindung der Formel

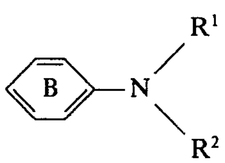

(XIV)

oder

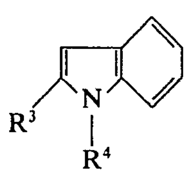

(XV)

in welchen

$R^1$, $R^2$, B, $R^3$ und $R^4$ die für die Formeln II und III angegebenen Bedeutungen haben,
und die Ringe die in den Formeln I, II und III angegebenen Substituenten enthalten können,

und mit einer Verbindung der Formel

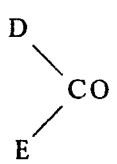

(XVI)

worin

D und E die bei Formel (I) angegebene Bedeutung haben,

in beliebiger Reihenfolge.

Die Kondensation mit (XIV) oder (XV) wird am zweckmäßigsten in Phosphoroxychlorid unter Zusatz von Phosphorpentoxid bei Temperaturen zwischen etwa 35°C und dem Siedepunkt der Mischung ausgeführt. Die Umsetzung von (XIII) und (XIV) ist beispielsweise aus der US-A-4 000 141 bekannt.

Die anschließende Kondensation mit (XVI) oder die Umsetzung von (XIII) mit (XVI) im Mol-Verhältnis 2 : 1 wird zweckmäßig in 65—96%iger Schwefelsäure oder in Mischungen aus Eisessig und Schwefelsäure im Temperaturbereich von 20—120°C durchgeführt.

Die neuen Farbstoffe besitzen eine sehr hohe Celluloseaffinität, die auch das Färben ligninfreier, gebleichter Zellstoffe unter nahezu quantitativer Farbstoffaufnahme ermöglicht. Die anfallenden Abwässer enthalten demzufolge keinen oder fast keinen Farbstoff. Die Färbungen zeichnen sich durch gute Lichtechtheiten aus.

Die Farbstoffe eignen sich auch zum Färben, Bedrucken und Massefärben von Materialien die überwiegend oder vollständig aus Polyacrylnitril oder seinen Mischpolymerisaten mit anderen Vinylmonomeren, wie Vinylidencyanid, Vinylidenchlorid, Vinylchlorid, Vinylacetat, Vinylalkohol, Acryl- oder Methacrylester, bestehen, oder aus sauer modifizierten Polyestern oder Polyamiden. Die erhaltenen Färbungen und Drucke zeichnen sich durch hohe Allgemeinechtheiten, insbesondere durch hohe Licht-, Naß-, und Schweißechtheiten, durch eine hohe Affinität zur Faser und durch eine hohe pH-Stabilität aus.

Obwohl die erfindungsgemäßen Farbstoffe gut wasserlöslich sind, bluten sie beim Ausbluttest spinngefärbter Polyacrylnitrilfasern nicht oder nur in sehr geringem Maße aus. Sie sind daher hervorragend zum Spinnfärben von Polyacrylnitril oder seinen Mischpolymerisaten geeignet. Die Löslichkeit der erfindungsgemäßen Farbstoffe in den zum Spinnen benutzten Lösungsmitteln ist gut, wenn sie als übliche Salze anorganischer Säuren, z. B. als Chlorid, Sulfat, Acetat oder Lactat, vorliegen. Die Löslichkeit kann durch Überführen der Farbstoffsalze in Salze mehrkerniger aromatischer Sulfonsäuren erhöht werden. Die Farbstoffe sind auch in Form ihrer Basen der Formeln Ia bzw. Ib ausgezeichnet zum Spinnfärben geeignet.

Gegenstand der Erfindung sind außerdem Naphtholactamderivate der Formel

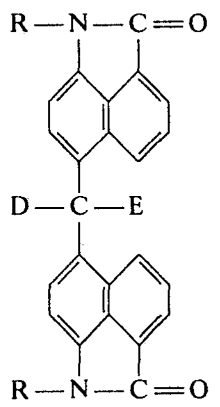

(XVII)

worin

R, D und E die obengenannte Bedeutung haben und der Naphthalinring in der beschriebenen Weise substituiert sein kann,

und ihre Herstellung aus den Verbindungen (XIII) und (XVI), insbesondere Formaldehyd.

Der Formaldehyd liegt dabei am zweckmäßigsten als Paraformaldehyd vor.

Andere geeignete Carbonylverbindungen (XVI) sind beispielsweise Acetaldehyd, Trichloracetaldehyd, Butanal-(1), Acrolein, Zimtaldehyd, Hydrozimtaldehyd, Benzaldehyd, o-Chlorbenzaldehyd, p-Chlorbenzaldehyd, p-Tolylaldehyd, Chromenon-3-aldehyd, Thiophen-2-aldehyd, Indol-3-aldehyd, Pyridin-3-aldehyd, Aceton und Cyclohexanon.

Geeignete Verbindungen der Formel (XIII) sind z. B. die im US-Patent 4 000 141 und in der DE-OS 2 557 503 genannten in p-Stellung zum Stickstoff unsubstituierten Naphtholactamderivate.

Durch Chlorieren bzw. Bromieren von aus (XIII) und (XVI) hergestellten Kondensationsprodukten erhält man weitere Verbindungen der Formel (XVII).

Geeignete Verbindungen der Formel (XIV) sind z. B. die im US-Patent 4 000 141 und in der DE-OS 2 557 503 genannten Tetrahydrochinoline und die im US-Patent 4 051 084 und in den Deutschen Offenlegungsschriften 2 255 058, 2 255 059, 2 255 060, 2 344 672, 2 344 735, 2 344 901 angeführten Kupplungskomponenten.

Als Anionen $An^-$ kommen z. B. die in der DE-OS 2 255 058 aufgezählten Anionen in Betracht.

Beispiel 1

81,3 g des Naphtholactamderivates der Formel

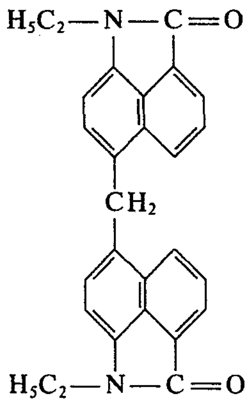

(XVII)

108,7 g N,β-Acetoxyethyl-2,2,4-trimethyl-tetrahydrochinolin und 150 g Phosphoroxychlorid werden verrührt, mit 25 g Phosphorpentoxid versetzt und 12 h bei 75°C gehalten. Dann rührt man die Schmelze in 1 l Wasser von 45°C ein und erhitzt die erhaltene Lösung zum Sieden. Nach dem Abkühlen kristallisiert der erhaltene Farbstoff. Es wird abgepreßt, in 1,5 l heißem Wasser gelöst, mit 20 g A-Kohle geklärt und dann mit Siedesalz wieder ausgefällt. Der kristalline Farbstoff wird abgenutscht und im Vakuum getrocknet. Er hat die Formel

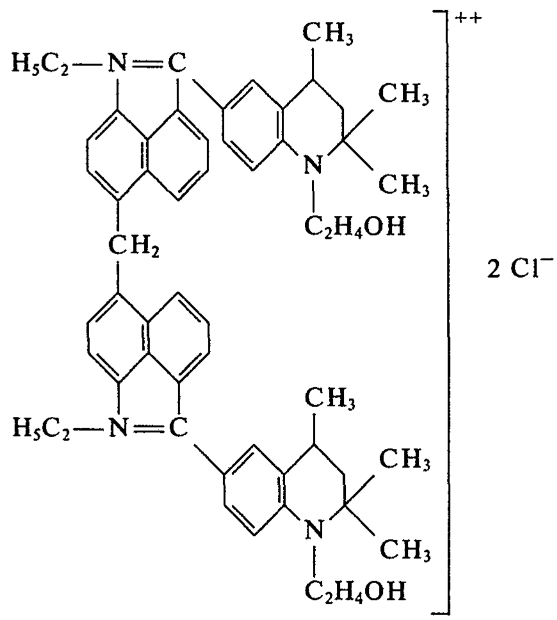

und färbt Dralon im Ausziehverfahren blau (C.I. Hue Ind. Chart Nr. 14).

Beispiel 2

30 g des in Beispiel 1 beschriebenen Farbstoffes werden in 270 g Dimethylformamid gelöst. Diese Lösung wird unmittelbar vor dem Verspinnen mittels einer Dosierpumpe in eine Lösung von 3 kg sauer modifiziertem Polyacrylnitril in Dimethylformamid eingebracht und dann die homogenisierte Lösung nach einem üblichen Trockenspinnverfahren versponnen. Man erhält intensiv rotstichig blaue Fäden, die sich durch ausgezeichnete Echtheiten, insbesondere Licht-, Reib-, Naß- und Dämpfechtheiten auszeichnen.

Führt man die Faserherstellung nach einem der üblichen Naßspinnverfahren durch, erhält man ein gleich gutes Ergebnis. Die Fäll- und Verstreckbäder werden nicht eingefärbt, weil der Farbstoff nicht ausblutet.

Beispiel 3

40,6 g des in Beispiel 1 verwendeten Naphtholactamderivates, 44,7 g N-Ethyl-2,2,4-trimethyltetrahydrochinolin und 75 g $POCL_3$ werden gemischt, mit 12,5 g Phosphorpentoxid versetzt und 12 h bei 75°C verrührt. Dann rührt man die Schmelze in 500 ml Wasser ein und rührt nach, bis das $POCl_3$ zersetzt und der Farbstoff kristallin ist. Es wird abgepreßt und im Vakuum getrocknet. Seine Konstitution entspricht der Formel:

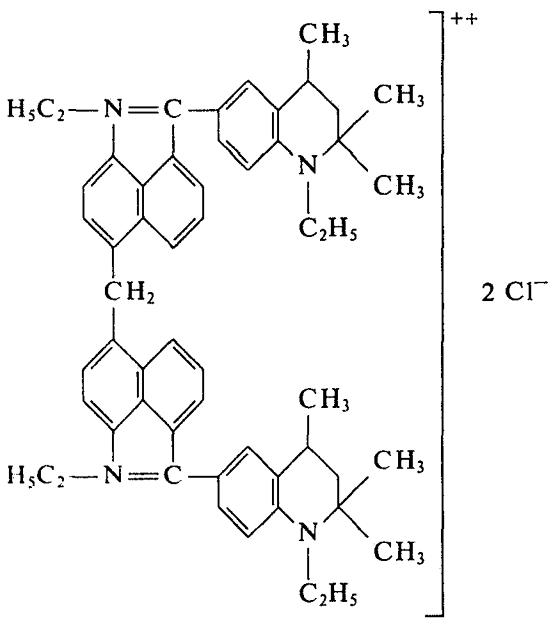

Setzt man diesen Farbstoff wie in Beispiel 2 beschrieben zum Spinnfärben ein, erhält man ebenfalls blaue Fäden mit hervorragenden Echtheiten. Im Ausziehverfahren erhält man auf DRALON ein grünstichiges Blau (C.I. Hue Indication Chart Nr. 15).

Beispiel 4

Ein aus gebleichtem Sulfitzellstoff bestehender Trockenstoff wird im Holländer mit soviel Wasser angeschlagen und bis zum Mahlgrad 40° SR gemahlen, daß der Trockengehalt etwas über 2,5% liegt, und anschließend mit Wasser auf exakt 2,5% Trockengehalt des Dickstoffes eingestellt. 200 g dieses Dickstoffes werden mit 5 g einer 0,25%igen wäßrigen Lösung des Farbstoffes gemäß Beispiel 3 versetzt, ca. 5 min verrührt und ohne Zusatz von Harzleim und Alaun zu Papier verarbeitet. Man erhält ein in kräftigem Blauton eingefärbtes Blattpapier. Das Abwasser enthält lt. fotometrischer Bestimmung nur ca. 1% des eingesetzten Farbstoffes. Erfolgt die Einfärbung der Papiermassen in Gegenwart von 2% Harzleim und 4% Alaun, so erhält man ein ähnliches Ergebnis.

Eine blaue Papierfärbung von erwünscht neutralem Blauton erhält man, wenn man die Färbung mit C.I. Basic Blue 140 durchführt, dem, auf Wirkstoff bezogen, 10% des Farbstoffes von Beispiel 3 zugesetzt wurden.

Setzt man statt des Farbstoffes des Beispiels 3 den Farbstoff des Beispiels 1 ein und verfährt in der in Beispiel 4 beschriebenen Weise, so erhält man ebenfalls gute Ergebnisse.

Beispiel 5

40,6 g des in Beispiel 1 verwendeten Naphtholactamderivates, 38,5 g 2,2,4-Trimethyl-tetrahydrochinolin und 95 g $POCl_3$ werden bei Raumtemperatur verrührt, mit 12,5 g $P_2O_5$ versetzt und bei 70°C 12 h lang kondensiert. Dann wird die Schmelze in 500 ml Wasser eingerührt. Der Farbstoff wird abgenutscht, nachdem das Phosphoroxychlorid restlos zersetzt ist. Der Farbstoff entspricht der Formel:

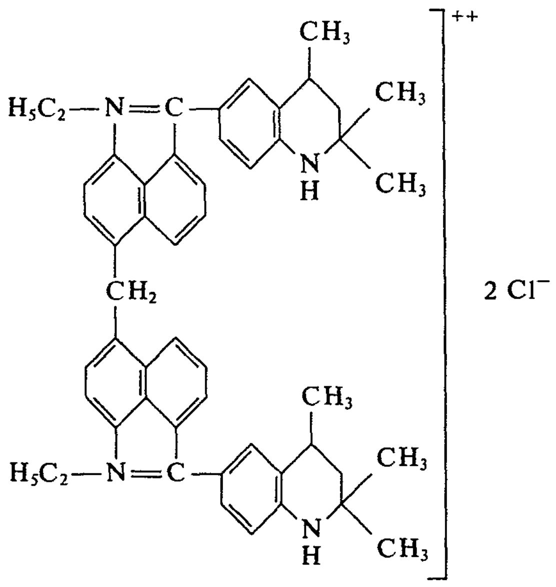

Setzt man den Farbstoff analog der in Beispiel 2 beschriebenen Arbeitsweise zum Spinnfärben ein, erhält man blaue Fäden mit sehr guten Echtheiten.

Bei der Verwendung als Papierfarbstoff gemäß Beispiel 4 erhält man eine blaue Färbung.

Der Farbstoff färbt Polyacrylnitril im Ausziehverfahren grünstichig blau (Hue Indication Chart Nr. 15).

Beispiel 6

8 g des in Beispiel 1 formelmäßig aufgeführten Naphtholactamderivates werden mit 10 g Diethylanilin, 30 g $POCl_3$ und 7 g $P_2O_5$ 15 h bei 40°C verrührt. Nach Zersetzen der Schmelze mit Wasser wird der Farbstoff mit Natriumchlorid gefällt, abgesaugt, in Wasser heiß gelöst, mit 3 g A-Kohle geklärt und mit Siedesalz wieder ausgefällt. Es wird abgesaugt und im Vakuum getrocknet. Der erhaltene Farbstoff entspricht der Formel:

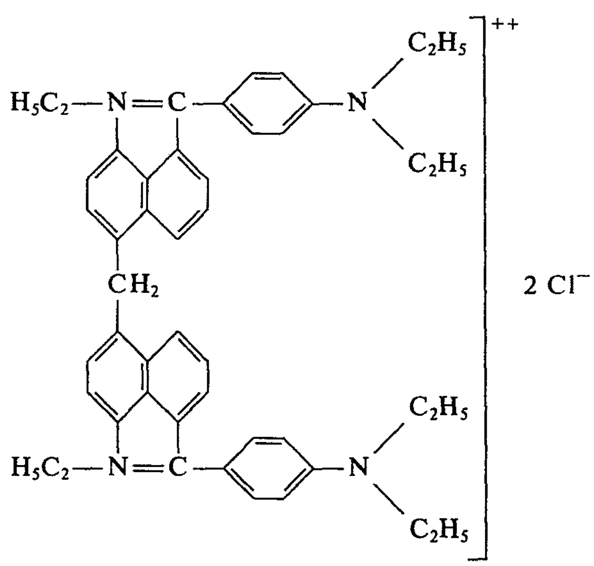

und ist gut zur Herstellung rotstichig blauer spinngefärbter Fäden analog Beispiel 2 geeignet.

Polyacrylnitril wird im Ausziehverfahren von dem Farbstoff blau gefärbt (C.I. Hue Ind. Chart Nr. 14).

Beispiel 7

4 g des in Beispiel 1 verwendeten Naphtholactamderivates 6 g 4,β-Acetoxyethyloxyethyl-N-β-ace-toxyethyl-diphenylamin, 25 g $POCl_3$ und 5 g $P_2O_5$ werden 15 h bei 40°C kondensiert. Die Schmelze wird mit Wasser zersetzt, die Mischung zum Sieden erhitzt und der auskristallisierte Farbstoff nach dem Abkühlen abgesaugt und getrocknet. Er hat die Formel:

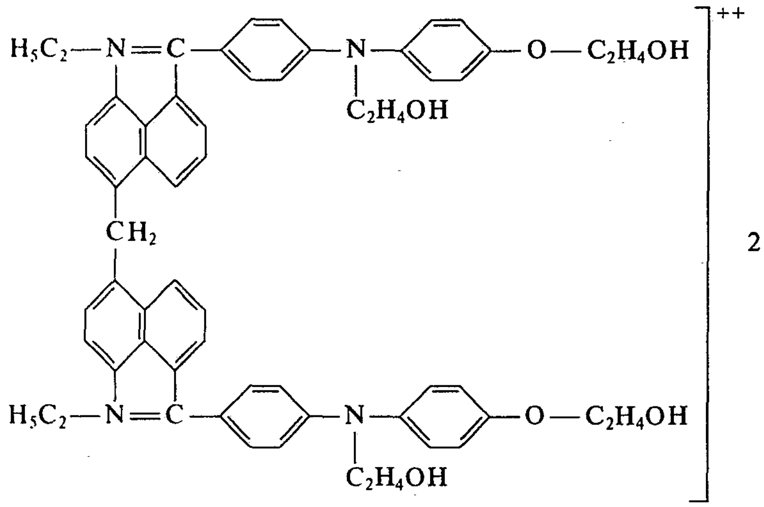

Setzt man ihn analog Beispiel 2 zum Spinnfärben von Polyacrylnitril ein, erhält man rotstichig blaue Fäden mit ausgezeichneten Echtheiten.

Färbt man Polyacrylnitrilgewebe mit dem vorstehend beschriebenen Farbstoff nach einem üblichen Ausziehverfahren, so erhält man eine rotstichig blaue Färbung mit ausgezeichneter Lichtechtheit (C.I. Hue Indication Chart Nr. 13).

Papier wird von diesem Farbstoff ebenfalls blau gefärbt.

Beispiel 8

8 g des in Beispiel 1 verwendeten Naphtholactamderivates werden mit 6 g 2-Methyl-indol in 5 g Phosphoroxychlorid bei 80°C kondensiert. Nach Zersetzen der Schmelze mit Wasser und Fällen mit Siedesalz erhält man den Farbstoff der Formel:

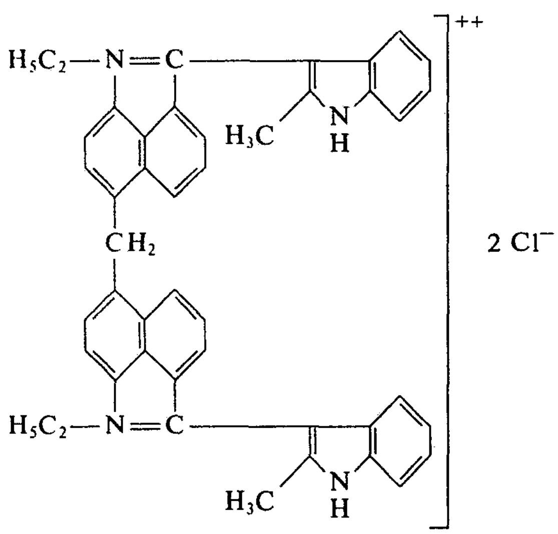

Er färbt sauer modifizierte Synthesefasern und Papier blaustichig violett (C.I. Hue Indication Chart Nr. 12).

Beispiel 9

Man verfährt wie in Beispiel 1 beschrieben, doch fällt man den Farbstoff aus der geklärten heißen Lösung nicht mit Siedesalz, sondern durch Eintropfen einer 40%igen Lösung des Ammoniumsalzes des Kondensationsproduktes aus Formaldehyd und Sulfonsäuregruppen enthaltendem 4,4'-Dioxydiphenylsulfon, bis das Farbsalz restlos ausgefallen ist. Man erhält ein in Wasser unlösliches blaues Pulver, welches nach dem Abpressen, Waschen mit Wasser und Trocknen hervorragend zum Spinnfärben, z.B. analog dem in Beispiel 2 angegebenen Verfahren, geeignet ist. Man erhält das gleiche Ergebnis wie in Beispiel 2.

Die in den Beispielen 3, 5, 6, 7 und 8 erwähnten Farbstoffe lassen sich aus ihren wäßrigen, evtl. mit A-Kohle geklärten Lösungen in gleicher Weise als in Wasser unlösliche, zum Spinnfärben hervorragend geeignete Salze des Kondensationsproduktes aus Formaldehyd und Sulfonsäuregruppen enthaltendem 4,4'-Dioxy-diphenylsulfon gewinnen.

Löst man diese in Wasser schwerlöslichen Salze in Dimethylformamid, welches 10% Essigsäure enthält, so ergeben sich im sichtbaren Bereich folgende Maxima:

Farbstoff aus Beispiel 1: 618 nm
Farbstoff aus Beispiel 3: 620 nm
Farbstoff aus Beispiel 5: 608 nm.

Beispiel 10

Man verfährt wie in Beispiel 1 beschrieben, doch fällt man den Farbstoff aus der geklärten, abgekühlten Lösung nicht mit Siedesalz sondern durch Eintropfen von Natronlauge, bis die Carbinolbase der Formel

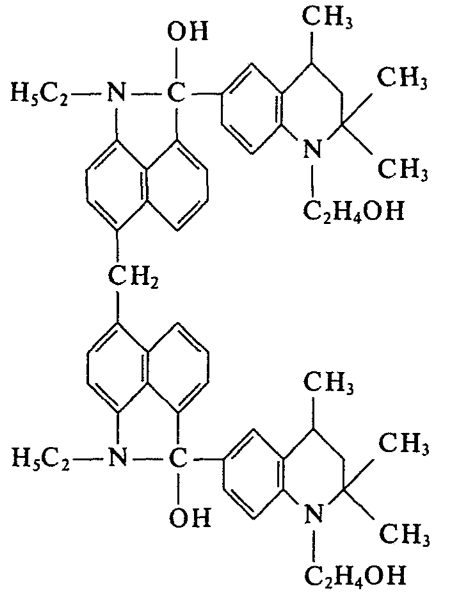

restlos ausgefallen ist. Sie ist nach dem Abpressen, Waschen mit Wasser und Trocknen hervorragend zum Spinnfärben analog dem in Beispiel 2 angegebenen Verfahren geeignet. Man erhält das gleiche Ergebnis wie in Beispiel 2.

Auch die Carbinolbasen der in den Beispielen 3, 5, 6, 7 und 8 erwähnten Farbstoffe sind ausgezeichnet zum Spinnfärben geeignet.

Analog der in den vorstehenden Beispielen angegebenen Arbeitsweise lassen sich z. B. auch folgende Farbstoffe herstellen. Sie ergeben auf Papier bzw. als Spinnfarbstoffe auch in Form ihrer Carbinolbasen oder als Fällungsprodukte mit mehrkernigen Sulfonsäuren Färbungen in den in der folgenden Tabelle angegebenen Farbtönen.

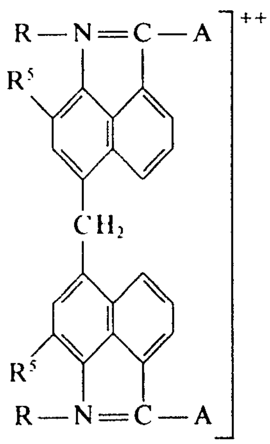

2 $An^-$

| Beispiel | R | A | $R^5$ | Färbung von Papier | H.I.C. Nr.*) | Spinnfärbung von PAC |
|---|---|---|---|---|---|---|
| 11 | $C_2H_5$ | 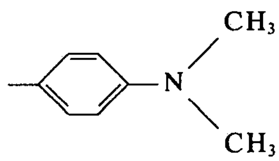 | H | blau | 13 | rost. blau |
| 12 | $CH_3$ | 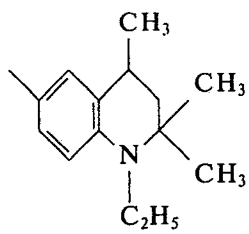 | H | blau | 14 | blau |
| 13 | $C_2H_5$ | desgl. | Cl | blau | 15 | blau |
| 14 | $C_2H_5$ | 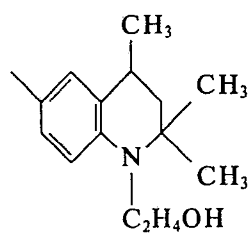 | Br | blau | 15 | blau |
| 15 | $C_2H_4CN$ | 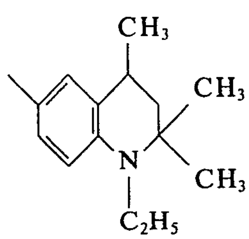 | H | rotst. blau | 13 | rotst. blau |
| 16 | $C_2H_4OH$ | desgl. | H | rotst. blau | 13 | rotst. blau |
| 17 | $-CH_2-COOCH_3$ | desgl. | H | blau | 13 | rotst. blau |
| 18 | $C_4H_9$ | desgl. | H | blau | 15 | blau |

Fortsetzung

| Beispiel | R | A | $R^5$ | Färbung von Papier | H.I.C. Nr.*) | Spinnfärbung von PAC |
|---|---|---|---|---|---|---|
| 19 | $C_2H_4OCH_3$ | 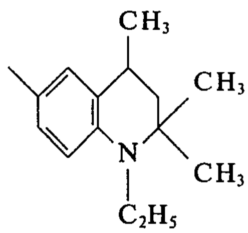 | H | blau | 14 | blau |
| 20 | $-CH_2-CH=CH_2$ | desgl. | H | blau | 15 | blau |
| 21 | $C_2H_4CN$ | 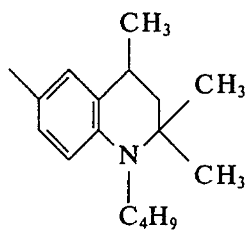 | H | blau | 15 | blau |
| 22 | $C_2H_5$ | 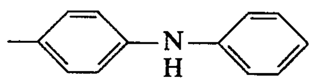 | H | blau | 13 | rotst. blau |
| 23 | $C_2H_5$ | 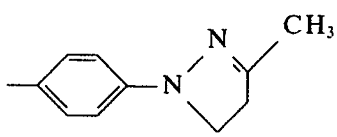 | H | blau | 14 | blau |
| 24 | $C_2H_5$ | 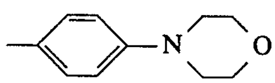 | H | blau | 13 | rotst. blau |
| 25 | $C_2H_5$ | 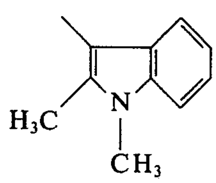 | H | violett | 12 | blaust. violett |
| 26 | $C_2H_5$ | 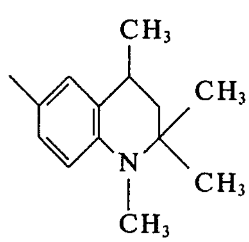 | H | blau | 14 | blau |
| 27 | $C_2H_5$ | 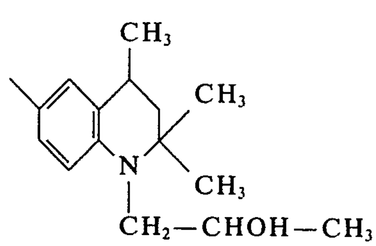 | H | blau | 15 | blau |

Fortsetzung

| Beispiel | R | A | $R^5$ | Färbung von Papier | H.I.C. Nr.*) | Spinnfärbung von PAC |
|---|---|---|---|---|---|---|
| 28 | $C_2H_5$ | 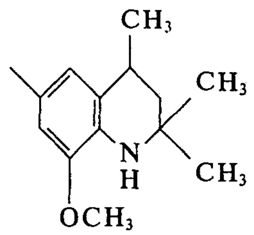 | H | blau | 15 | blau |
| 29 | $C_2H_5$ | 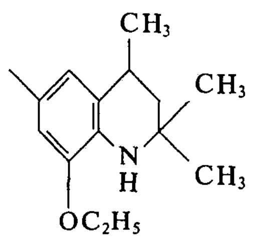 | H | blau | 15 | blau |
| 30 | $C_2H_5$ | 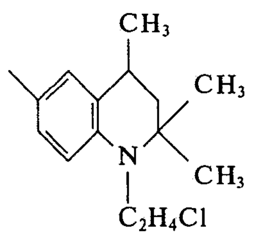 | H | blau | 14 | blau |
| 31 | $C_2H_5$ | 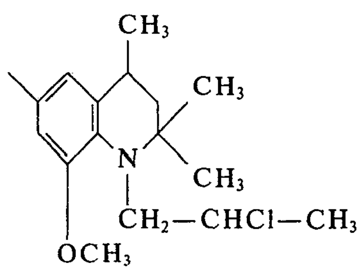 | H | blau | 16 | grünst. blau |
| 32 | $C_2H_5$ | 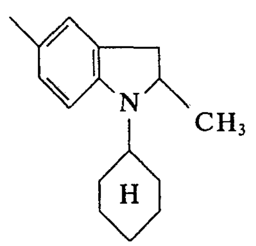 | H | blau | 13 | rotst. blau |

*) C.I. Hue Indication Chart Nr. beim Färben auf Polyacrylnitril nach dem Ausziehverfahren.

Beispiel 33

In 2 l Eisessig suspendiert man 394 g N-Ethyl-naphtholactam und gibt 36 g Paraformaldehyd zu und tropft bei 20—25°C unter Kühlen 200 ml konzentrierte Schwefelsäure ein. Dann wird bei dieser Temperatur 2—3 h nachgerührt und der Niederschlag abgepreßt. Man rührt ihn anschließend mit 1200 ml Methanol aus, preßt ab, wäscht mit Methanol nach und trocknet bei 50°C im Vakuum. Man erhält 391,9 g Methylen-bis-naphtholactam der Formel:

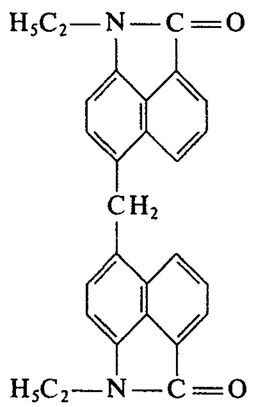

entsprechend 96,5% der Theorie. Schmelzpunkt 262°C; m/e = 406 ($M^{\oplus}$).
Löst man die Farbstoffe aus folgenden Beispielen in einer Mischung aus $^2/_3$ Methanol und $^1/_3$ 20%iger Essigsäure, werden im sichtbaren Bereich folgende Maxima gemessen:

| Farbstoff aus Beispiel | max/nm |
|---|---|
| 1 | 618 |
| 5 | 606 |
| 6 | 606 |
| 7 | 588 |
| 8 | 546 |
| 11 | 594 |
| 22 | 584 |
| 23 | 612 |
| 25 | 552 |

Beispiel 34

Man trägt 197 g N-Ethyl-naphtholactam in 1700 g 78%ige Schwefelsäure ein, fügt 74 g Benzaldehyd hinzu und erhitzt die Mischung 7 Stunden auf 100°C. Nach dem Abkühlen auf 25°C trägt man die Lösung in 5 l Eiswasser ein. Nach 1stündigem Verrühren wird der kristalline Niederschlag abgesaugt, mit Wasser gewaschen, 30 Minuten mit 5 l Wasser und soviel 50%iger Natronlauge, daß die Suspension schwach alkalisch reagiert, verrührt, wiederum mit Wasser gewaschen und bei 60°C im Vakuum getrocknet. Man erhält 232 g Verbindung der Formel

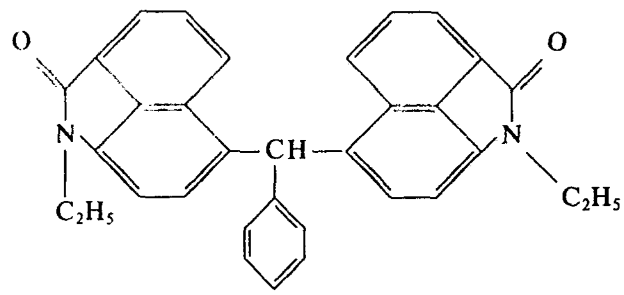

Schmelzpunkt: 274—275°C; m/e = 482 ($M^{\oplus}$).

Analoge Bis-naphtholactam-Verbindungen werden erhalten, wenn man anstelle von Benzaldehyd äquivalente Mengen eines der folgenden Aldehyde einsetzt (Schmelzpunkt des jeweiligen Bisnaphtholactams in Klammern): 2-Chlorbenzaldehyd (178°C), 4-Chlorbenzaldehyd (270°C), 4-Toluolaldehyd (157°C), Hydrozimtaldehyd (182°C), Chromen-4-on-3-aldehyd (207°C); ferner Pyridin-4-aldehyd, Furfural, Thiophen-2-aldehyd, Butanal-(1) und 4-Methoxybenzaldehyd.

Beispiel 35

24,2 g des im Beispiel 34 beschriebenen Naphtholactamderivates und 22,5 g N-Ethyl-2,2,4-trimethyl-tetrahydrochinolin werden in 40 g Phosphoroxychlorid verrührt, mit 7 g Phosphorpentoxid versetzt und dann 12 h bei 75°C kondensiert. Dann rührt man die Schmelze in 500 ml Wasser ein, rührt nach bis der Farbstoff kristallin ist, preßt ab und trocknet im Vakuum bei 50°C. Das erhaltene Produkt hat die Formel

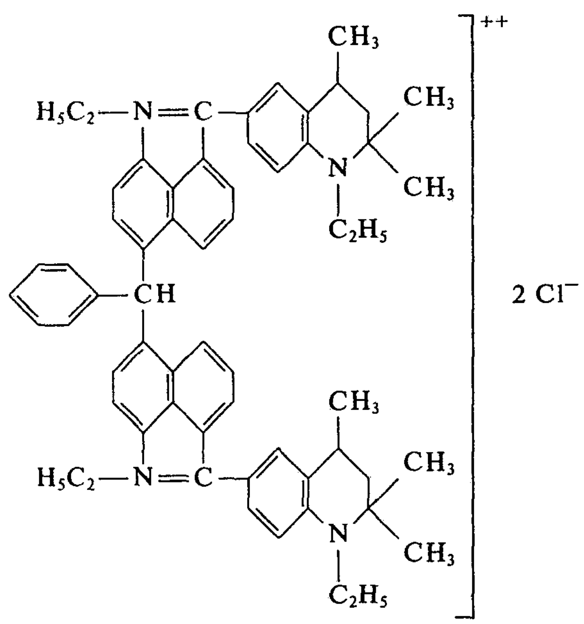

Es färbt Papier in einem brillanten Blau. Die Färbung auf Polyacrylnitril entspricht C. I. Hue Indication Chart. Nr. 14.

Beispiel 36

Man arbeitet zunächst wie in Beispiel 35, doch ersetzt man das N-Ethyl-2,2,4-trimethyltetrahydrochinolin durch die äquivalente Menge N-$\beta$-Acetoxyethyl-2,2,4-trimethyltetrahydrochinolin. Nach Ende der Kondensation rührt man die Schmelze in 350 ml Wasser von 45°C ein, erhitzt die Mischung zum Sieden und filtriert den erhaltenen Farbstoff nach dem Erkalten ab. Er wird in verdünnter Essigsäure gelöst, mit A-Kohle geklärt und mit Siedesalz wieder ausgefällt. Der Farbstoff entspricht der Formel

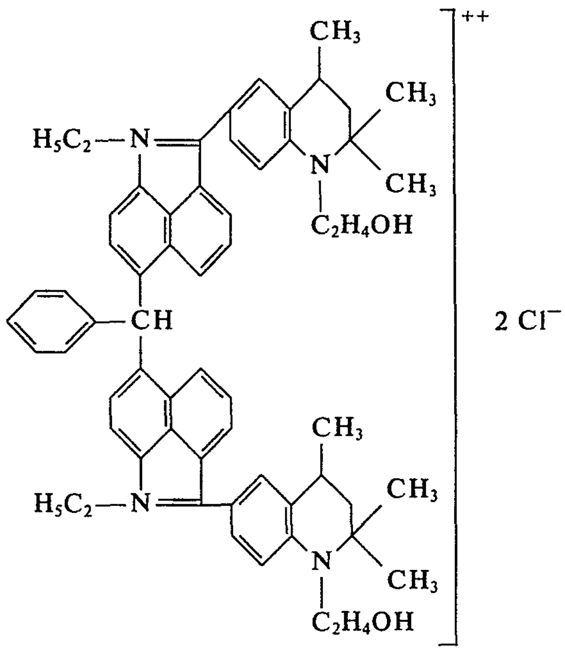

Er färbt Papier in einem brillanten Blau. Die Färbung auf Polyacrylnitril entspricht C. I. Hue Ind. Chart Nr. 14.

Setzt man analog Beispielen 34 und 35 folgende Naphtholactamderivate der Formel XVII und Tetrahydrochinoline der Formel XII miteinander um, erhält man die in folgender Tabelle aufgeführten Farbstoffe der Formel

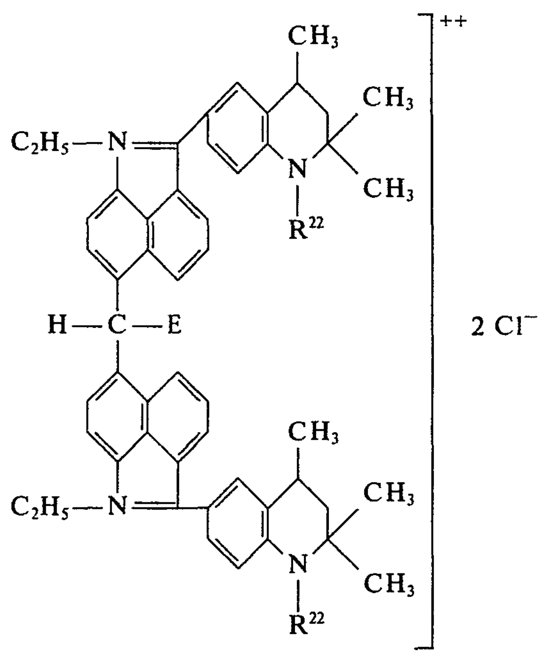

Tabelle

| Beispiel | E | $R^{21}$ | $R^{22}$ | Farbe auf Papier | Hue Ind. Ch. auf Polyacrylnitril |
|---|---|---|---|---|---|
| 36 | 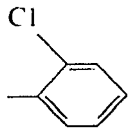 | $C_2H_4OCOCH_3$ | $C_2H_4OH$ | brill. Blau | 14 |
| 37 | desgl. | $C_2H_5$ | $C_2H_5$ | brill. Blau | 15 |
| 38 | 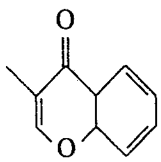 | $C_2H_4OCOCH_3$ | $C_2H_4OH$ | blau | 16 |
| 39 | desgl. | $C_2H_5$ | $C_2H_5$ | blau | 17 |
| 40 | 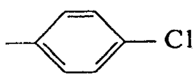 | $C_2H_4OCOCH_3$ | $C_2H_4OH$ | blau | 14 |
| 41 | 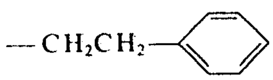 | desgl. | desgl. | blau | 14 |
| 42 | 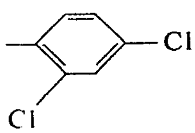 | $C_2H_5$ | $C_2H_5$ | blau | 15 |
| 43 | desgl. | $C_2H_4OCOCH_3$ | $C_2H_4OH$ | blau | 15 |
| 44 | 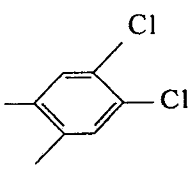 | $C_2H_5$ | $C_2H_5$ | blau | 46 |
| 45 | desgl. | $C_2H_4OCOCH_3$ | $C_2H_4OH$ | blau | 15 |
| 46 | 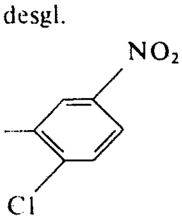 | $C_2H_5$ | $C_2H_5$ | blau | 15 |
| 47 | desgl. | $C_2H_4OCOCH_3$ | $C_2H_4OH$ | blau | 15 |

## Patentansprüche

1. Kationische Farbstoffe der allgemeinen Formel

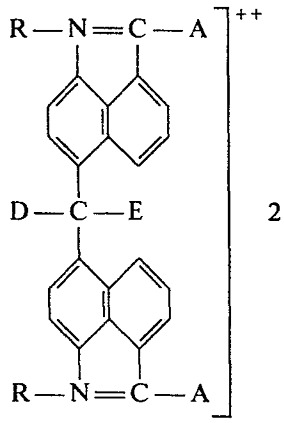

2 $An^-$ (I)

sowie ihre Basen

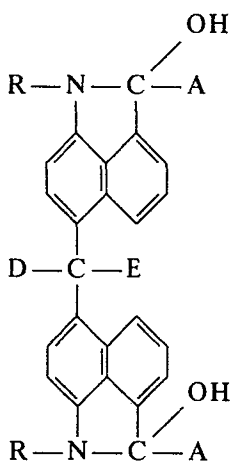

(Ia) bzw.

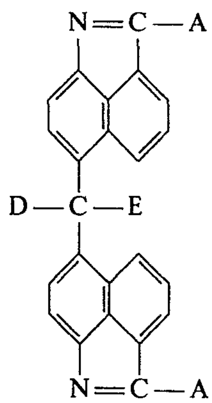

(Ib)

worin

R für Wasserstoff oder einen gegebenenfalls durch nichtionische Reste substituierten $C_1$- bis $C_8$-Alkylrest,

A für einen Rest der Formeln

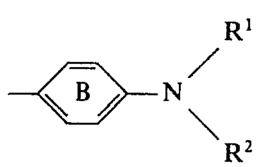

oder

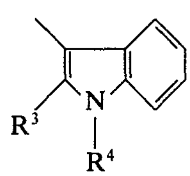

worin

$R^1$ Wasserstoff, einen gegebenenfalls durch nichtionische Reste substituierten $C_1$- bis $C_4$-Alkylrest oder Cycloalkyl und

$R^2$ einen gegebenenfalls durch nichtionische Reste substituierten $C_1$- bis $C_6$-Alkylrest, der gegebenenfalls einen Ring zum Ring B schließen kann, oder einen Aralkyl- oder Arylrest darstellen, oder

$R^1$ und $R^2$ zusammen mit dem N-Atom einen Ring bilden,

$R^3$ einen $C_1$- bis $C_4$-Alkylrest, einen $C_1$- bis $C_4$-Alkoxycarbonylrest oder einen Arylrest,

$R^4$ Wasserstoff oder einen gegebenenfalls durch nichtionische Reste substituierten $C_1$- bis $C_4$-Alkylrest bedeuten,

D und E für Wasserstoff, einen gegebenenfalls durch nichtionische Reste substituierten $C_1$- bis $C_4$-Alkyl- oder $C_2$- bis $C_4$-Alkenylrest und D zusätzlich für Aryl, Aralkyl, Aralkenyl oder einen heterocyclischen Rest stehen, oder D und E zusammen mit dem C-Atom einen Ring bilden, und

$An^-$ für ein Anion steht,

und worin die Ringe in der Farbstoffchemie übliche nichtionische Reste enthalten können.

2. Kationische Farbstoffe der allgemeinen Formel

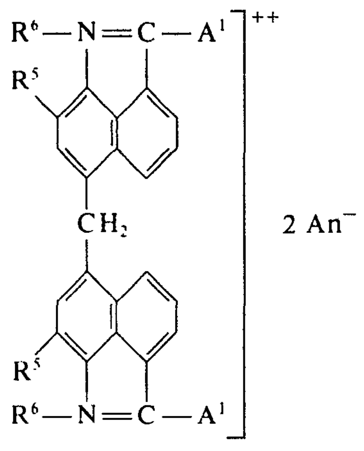

worin

$R^6$ für Wasserstoff, $C_1$- bis $C_4$-Alkyl, das durch Cyan, Hydroxy, $C_1$- bis $C_4$-Alkoxy, Chlor, $C_1$- bis $C_4$-Alkoxycarbonyl, Aminocarbonyl oder Mono- oder Di-$C_1$- bis $C_4$-alkylaminocarbonyl substituiert sein kann,

$R^5$ für Wasserstoff, Chlor oder Brom,

$A^1$ für

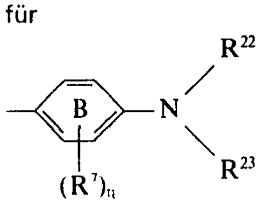

worin

$R^{22}$ Wasserstoff oder einen gegebenenfalls durch 1—3 Hydroxy, 1—3 Halogen, $C_1$- bis $C_4$-Alkoxy, Cyan, $C_1$- bis $C_4$-Alkylcarbonyloxy, Hydroxy-$C_1$- bis $C_4$-alkoxy, Aminocarbonyl, Carboxy, $C_1$- bis $C_4$-Alkoxycarbonyl oder $C_3$- oder $C_4$-Alkenyloxy, durch Acyloxy, Benzyloxy, Phenyloxy, Sulfonamido oder Acylamido substituierten $C_1$- bis $C_6$-Alkylrest, gegebenenfalls durch Halogen substituierten $C_2$- bis $C_4$-Alkenylrest oder gegebenenfalls durch $C_1$- bis $C_4$-Alkylrest substituierten Cyclohexylrest,

$R^{23}$ einen gegebenenfalls wie $R^{22}$ substituierten $C_1$- bis $C_6$-Alkylrest, Benzyl oder Phenyl, oder zusammen mit dem Ring B ein Tetrahydrochinolin, Indolin, Hexahydrocarbazol oder Dihydrobenzoxazin, die $C_1$- bis $C_4$-Alkyl-und Phenylgruppen tragen können, oder

$R^{22}$ und $R^{23}$ zusammen einen Pyrrolidin-, Piperidin-, Morpholin-, Pyrazolin- oder Piperazinring, die $C_1$- bis $C_4$-Alkyl- und Phenylgruppen tragen können,

$R^7$ $C_1$- bis $C_4$-Alkyl, $C_1$- bis $C_4$-Alkoxy oder Halogen und

n 1 bis 4 bedeuten,

oder für

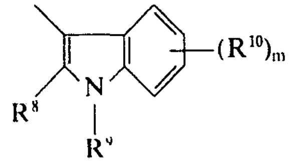

(VI)

worin

$R^8$ einen Alkylrest mit 1 bis 4 C-Atomen, einen Phenylrest oder einen Alkoxycarbonylrest mit 1 bis 4 C-Atomen,

$R^9$ Wasserstoff, einen gegebenenfalls durch Hydroxy, Halogen, Alkoxy mit 1 bis 4 C-Atomen, Cyan oder Acyloxy substituierten Alkylrest mit 1 bis 4 C-Atomen und

$R^{10}$ Wasserstoff, Halogen, $C_1$- bis $C_4$-Alkyl, $C_1$- bis $C_4$-Alkoxy, Carbalkoxy mit 1 bis 4 C-Atomen, $C_1$- bis $C_4$-Alkylsulfonyl, Phenylsulfonyl, Acetyl oder Benzoyl und

m 1 bis 4 bedeuten, und

$An^-$ für ein Anion stehen, und worin die Phenylreste durch $C_1$- bis $C_4$-Alkyl, $C_1$- bis $C_4$-Alkoxy, Hydroxy, Chlor oder Brom substituiert sein können.

3. Farbstoffe gemäß Anspruch 2, worin

$R^6$ für Wasserstoff, $C_1$- bis $C_4$-Alkyl, $\beta$-Cyanethyl, $\beta$-Hydroxyethyl, $\beta$-Alkoxyethyl, $\beta$-Chlorethyl, Mehoxycarbonylethyl, Ethoxycarbonylethyl, Methoxycarbonylmethyl oder Ethoxycarbonylmethyl,

$R^5$ für Wasserstoff und

$A^1$ für

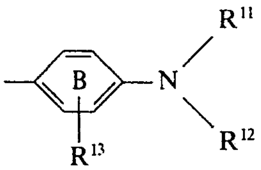

worin

$R^{11}$ Wasserstoff oder einen gegebenenfalls durch Hydroxy, Methoxy, Ethoxy, Chlor, Cyan, Acetoxy oder Acetyl substituierten $C_1$- bis $C_4$-Alkylrest,

$R^{12}$ einen gegebenenfalls durch Hydroxy, Methoxy, Ethoxy, Chlor, Cyan, Acetoxy oder Acetyl substituierten $C_1$- bis $C_4$-Alkylrest, Benzyl oder gegebenenfalls durch Chlor, Methyl, Methoxy oder Ethoxy substituiertes Phenyl,

$R^{13}$ Wasserstoff, Methyl, Chlor, Methoxy oder Ethoxy bedeuten,

für

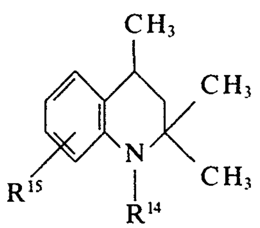

worin

$R^{14}$ Wasserstoff oder $C_1$- bis $C_4$-Alkyl, $C_2$- bis $C_4$-Hydroxyalkyl, Chlorethyl, Bromethyl, Methoxyethyl, Cyanethyl, Acetoxyethyl, Hydroxyethoxyethyl, Aminocarbonylethyl, Carboxyethyl, $\gamma$-Cyanpropyl, $\beta$-Hydroxy-$\gamma$-allyloxy-n-propyl, $\beta$-Hydroxy-$\gamma$-methoxy-n-propyl, $\beta$-Hydroxy-$\gamma$-ethoxy-n-propyl, $\beta$-Hydroxy-$\gamma$-butoxy-n-propyl, $\beta$-Chlor-n-propyl, $\beta$-Chlor-n-butyl, $\beta$-Chlor-i-butyl, $\beta,\gamma$-Dichlor-n-propyl, $\beta$-Acetoxy-n-propyl, $\beta$-Hydroxy-$\gamma$-chlor-n-propyl, Vinyl, Allyl, Methallyl, Chlorallyl oder Cyclohexyl und

$R^{15}$ Wasserstoff, Methoxy oder Ethoxy bedeuten,

oder für

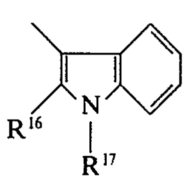

worin

$R^{16}$ Methyl oder gegebenenfalls durch Chlor, Methyl oder Methoxy substituiertes Phenyl und

$R^{17}$ einen gegebenenfalls durch Hydroxy, Chlor, Cyan oder Acyloxy substituierten $C_1$- bis $C_4$-Alkylrest oder Wasserstoff bedeuten,

stehen.

4. Farbstoffe der allgemeinen Formel

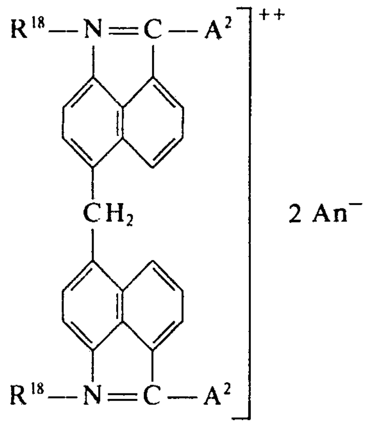

worin

$R^{18}$ für Methyl, Ethyl oder $\beta$-Cyanethyl und
$A^2$ für

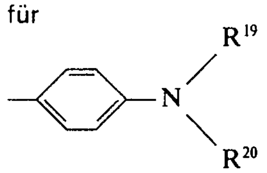

worin

$R^{19}$ Wasserstoff, Methyl, Ethyl, Cyanethyl, Hydroxyethyl, Acetoxyethyl, Methoxyethyl, Ethoxyethyl, Chlorethyl, Hydroxypropyl, Propyl oder Butyl und
$R^{20}$ eine der für $R^{19}$ genannten Alkylgruppen oder Phenyl, Methoxyphenyl, Ethoxyphenyl, Methylphenyl oder Chlorphenyl bedeuten,

oder ganz besonders bevorzugt für

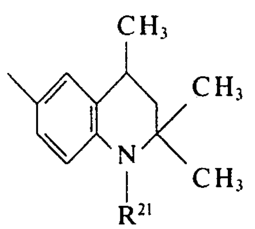

worin

$R^{21}$ Wasserstoff, $C_1$- bis $C_4$-Alkyl, Hydroxyethyl, Hydroxypropyl, Cyanethyl, Chlorethyl, Acetoxyethyl, Acetoxypropyl, Methoxyethyl, Ethoxyethyl, Hydroxybutyl, Hydroxycarbonylethyl oder Hydroxyethoxyethyl bedeutet, und

$An^-$ für ein Anion stehen.

5. Verfahren zur Herstellung von Farbstoffen gemäß Anspruch 1, dadurch gekennzeichnet, daß man Naphtholactamderivate der Formel

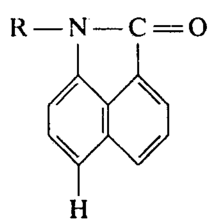

worin

R die Bedeutung von Anspruch 1 hat,

und die Naphtholactamringe wie in Anspruch 1 angegeben substituiert sein können,

mit einer Verbindung der Formel

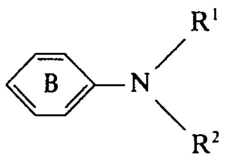

oder

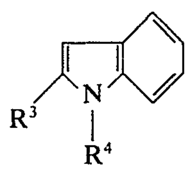

in welchen

$R^1$, $R^2$, B, $R^3$ und $R^4$ die in Anspruch 1 angegebene Bedeutung haben,
die Ringe die in Anspruch 1 angegebenen Substituenten enthalten können,

und mit einer Verbindung der Formel

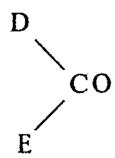

in der

D und E die in Anspruch 1 angegebene Bedeutung haben, in beliebiger Reihenfolge kondensiert.

6. Methylen-bis-naphtholactamverbindungen der allgemeinen Formel

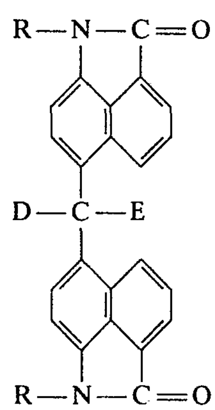

worin

R, D und E die in Anspruch 1 angegebene Bedeutung haben und

die Naphtholactamringe in der in Anspruch 1 angegebenen Weise substituiert sein können.

7. Verfahren zur Herstellung von Methylen-bis-naphtholactamverbindungen gemäß Anspruch 6, dadurch gekennzeichnet, daß man Naphtholactamverbindungen der Formel

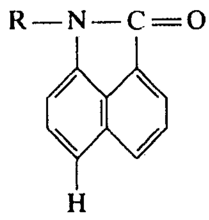

worin

R die Bedeutung von Anspruch 1 hat, und

der Naphtholactamring wie in Anspruch 1 angegeben substituiert sein kann,

mit Verbindungen der Formel

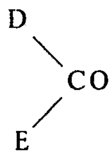

worin

E und D die Bedeutung von Anspruch 1 haben, insbesondere mit Formaldehyd, kondensiert.

8. Verfahren zum Färben von kationisch färbbaren Fasern aus Polymerisaten und Mischpolymerisaten des Acrylnitrils und des Dicyanethylens sowie von sauer modifizierten Fasern aus Polyamiden und Polyestern, dadurch gekennzeichnet, daß man Farbstoffe gemäß Ansprüchen 1 bis 4 verwendet.

9. Verfahren zum Färben von Papier, dadurch gekennzeichnet, daß man Farbstoffe gemäß Ansprüchen 1 bis 4 verwendet.

10. Verfahren zum Spinnfärben von Polyacrylnitril und dessen Mischpolymerisaten, dadurch gekennzeichnet, daß man Farbstoffe gemäß Ansprüchen 1 bis 4 verwendet.

## Claims

1. Cationic dyestuffs of the general formula

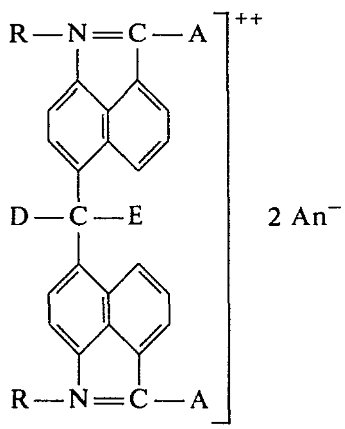

(I)

and their bases

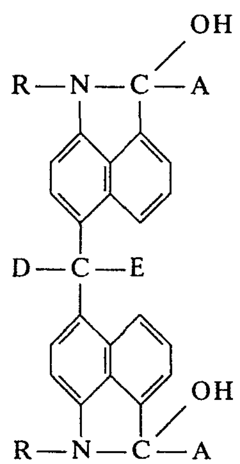

(Ia)

or

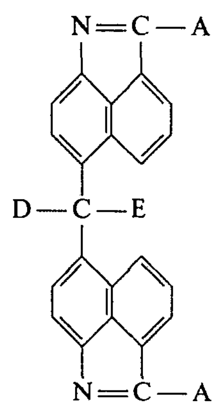

(Ib)

wherein

R represents hydrogen or a $C_1$- to $C_8$-alkyl radical optionally substituted by nonionic radicals,

A represents a radical of the formula

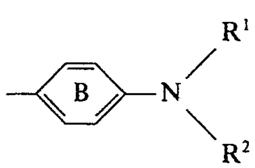

or

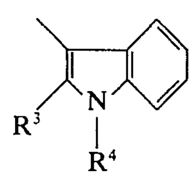

wherein

$R^1$ represents hydrogen, a $C_1$- to $C_4$-alkyl radical optionally substituted by nonionic radicals or cycloalkyl and

$R^2$ represents a $C_1$- to $C_6$-alkyl radical optionally substituted by nonionic radicals and optionally capable of closing a ring to ring B or an aralkyl or aryl radical or

$R^1$ and $R^2$ together with the N atom, form a ring,

$R^3$ denotes a $C_1$- to $C_4$-alkyl radical, a $C_1$- to $C_4$-alkoxycarbonyl radical or an aryl radical,

$R^4$ denotes hydrogen or a $C_1$- to $C_4$-alkyl radical optionally substituted by nonionic radicals,

D and E represent hydrogen, a $C_2$- to $C_4$-alkenyl or $C_1$- to $C_4$-alkyl radical optionally substituted by nonionic radicals and D additionally represents aryl, aralkyl, aralkenyl or a heterocyclic radical, or D and E, together with the C atom, form a ring, and

An represents an anion,

and wherein the rings can contain nonionic radicals customary in dyestuff chemistry.

2. Cationic dyestuffs of the general formula

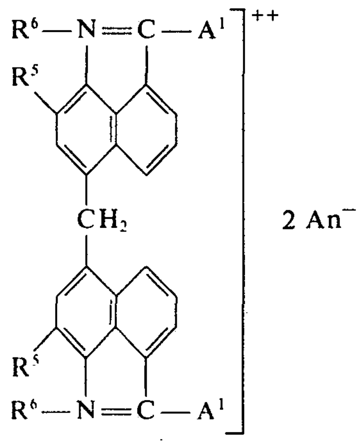

wherein

$R^6$ represents hydrogen, $C_1$- to $C_4$-alkyl which can be substituted by cyano, hydroxyl, $C_1$- to $C_4$-alkoxy, chlorine, $C_1$- to $C_4$-alkoxycarbonyl, aminocarbonyl or mono- or di-$C_1$- to $C_4$-alkylaminocarbonyl,

$R^5$ represents hydrogen, chlorine or bromine,

$A^1$ represents

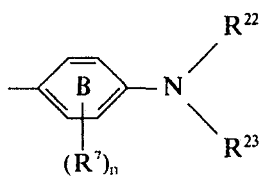

wherein

$R^{22}$ denotes hydrogen or a $C_1$- to $C_6$-alkyl radical optionally substituted by 1—3 hydroxyl, 1—3 halogen, $C_1$- to $C_4$-alkoxy, cyano, $C_1$- to $C_4$-alkylcarbonyloxy, hydroxy-$C_1$- to $C_4$-alkoxy, aminocarbonyl, carboxyl, $C_1$- to $C_4$-alkoxycarbonyl or $C_3$- to $C_4$-alkenyloxy, by acyloxy, benzyloxy, phenyloxy, sulphonamido or acylamido, a $C_2$- to $C_4$-alkenyl radical optionally substituted by halogen or a cyclohexyl radical optionally substituted by a $C_1$- to $C_4$-alkyl radical,

$R^{23}$ denotes a $C_1$- to $C_6$-alkyl radical optionally substituted like $R^{22}$, benzyl or phenyl, or, together with the ring B, a tetrahydroquinoline, indoline, hexahydrocarbazole or dihydrobenzoxazine, all of which can carry $C_1$- to $C_4$-alkyl and phenyl groups, or

$R^{22}$ and $R^{23}$ together denote a pyrrolidine, piperidine, morpholine, pyrazoline or piperazine ring, all of which can carry $C_1$- to $C_4$-alkyl and phenyl groups,

$R^7$ denotes $C_1$- to $C_4$-alkyl, $C_1$- to $C_4$-alkoxy or halogen and

n denotes 1 to 4,

or $A^1$ represents

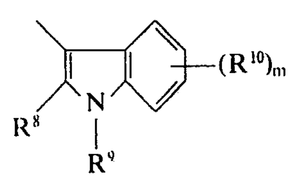

(VI)

wherein

$R^8$ denotes an alkyl radical having 1 to 4 C atoms, a phenyl radical or an alkoxycarbonyl radical having 1 to 4 C atoms,

$R^9$ denotes hydrogen, an alkyl radical having 1 to 4 C atoms and optionally substituted by hydroxyl, halogen, alkoxy having 1 to 4 C atoms, cyano or acyloxy and

$R^{10}$ denotes hydrogen, halogen, $C_1$- to $C_4$-alkyl, $C_1$- to $C_4$-alkoxy, carbalkoxy having 1 to 4 C atoms, $C_1$- to $C_4$-alkylsulphonyl, phenylsulphonyl, acetyl or benzoyl and

m denotes 1 to 4, and

$An^-$ represents an anion, and wherein the phenyl radicals can be substituted by $C_1$- to $C_4$-alkyl, $C_1$- to $C_4$-alkoxy, hydroxyl, chlorine or bromine.

3. Dyestuffs according to Claim 2, wherein

$R^6$ represents hydrogen, $C_1$- to $C_4$-alkyl-β-cyanoethyl, β-hydroxyethyl, β-alkoxyethyl, β-chloroethyl, methoxycarbonylethyl, ethoxycarbonylethyl, methoxycarbonylmethyl or ethoxycarbonylmethyl,

$R^5$ represents hydrogen and

$A^1$ represents

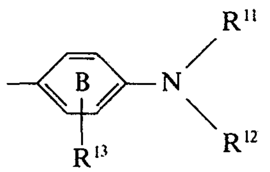

wherein

$R^{11}$ denotes hydrogen or a $C_1$- to $C_4$-alkyl radical optionally substituted by hydroxyl, methoxy, ethoxy, chlorine, cyano, acetoxy or acetyl,

$R^{12}$ denotes a $C_1$- to $C_4$-alkyl radical optionally substituted by hydroxyl, methoxy, ethoxy, chlorine, cyano, acetoxy or acetyl, benzyl or phenyl optionally substituted by chlorine, methyl, methoxy or ethoxy,

$R^{13}$ denotes hydrogen, methyl, chlorine, methoxy or ethoxy,

or $A^1$ represents

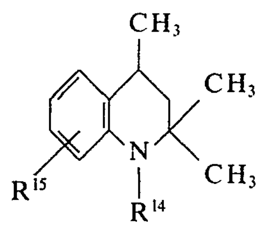

wherein

$R^{14}$ denotes hydrogen or $C_1$- to $C_4$-alkyl, $C_2$- to $C_4$-hydroxyalkyl, chloroethyl, bromoethyl, methoxyethyl, cyanoethyl, acetoxyethyl, hydroxyethoxyethyl, aminocarbonylethyl, carboxy ethyl, γ-cyanopropyl, β-hydroxy-γ-allyloxy-n-propyl, β-hydroxy-γ-methoxy-n-propyl, β-hydroxy-γ-ethoxy-n-propyl, β-hydroxy-γ-butoxy-n-propyl, β-chloro-n-propyl, β-chloro-n-butyl, β-chloro-i-butyl, β,γ-dichloro-n-propyl, β-acetoxy-n-propyl, β-hydroxy-γ-chloro-n-propyl, vinyl, allyl, metallyl, chloroallyl or cyclohexyl and

$R^{15}$ denotes hydrogen, methoxy or ethoxy,

or $A^1$ represents

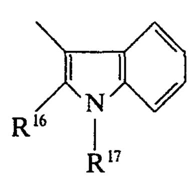

wherein

$R^{16}$ denotes methyl or phenyl optionally substituted by chlorine, methyl or methoxy and
$R^{17}$ denotes a $C_1$- to $C_4$-alkyl, radical optionally substituted by hydroxyl, chlorine, cyano or acyloxy or hydrogen.

4. Dyestuffs of the general formula

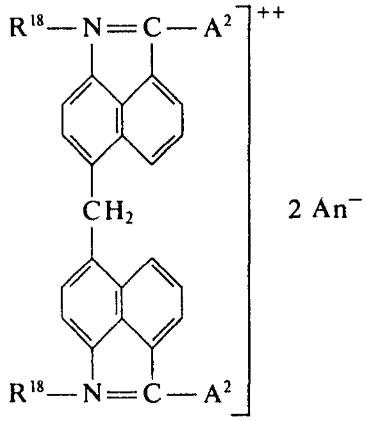

wherein

$R^{18}$ represents methyl, ethyl or $\beta$-cyanoethyl and
$A^2$ represents

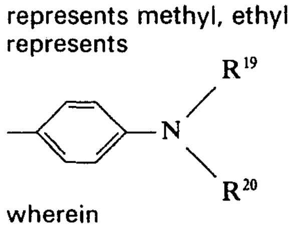

wherein

$R^{19}$ denotes hydrogen, methyl, ethyl, cyanoethyl, hydroxyethyl, acetoxyethyl, methoxyethyl, ethoxyethyl, chloroethyl, hydroxypropyl, propyl or butyl and
$R^{20}$ denotes one of the alkyl groups mentioned for
$R^{19}$ or phenyl, methoxyphenyl, ethoxyphenyl, methylphenyl or chlorophenyl or
$A^2$ very particularly preferably represents

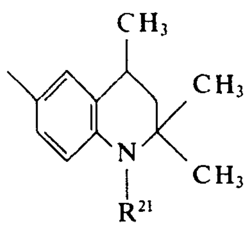

wherein

$R^{21}$ denotes hydrogen, $C_1$- to $C_4$-alkyl, hydroxyethyl, hydroxypropyl, cyanotehyl, chloroethyl, acetoxyethyl, acetoxypropyl, methoxyethyl, ethoxyethyl, hydroxybutyl, hydroxycarbonylethyl or hydroxyethoxyethyl and

$An^-$ represents an anion.

5. Process for preparing dyestuffs according to Claim 1, characterised in that naphtholactam derivatives of the formula

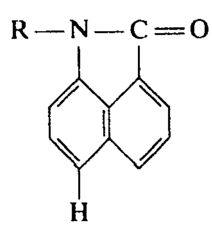

wherein

R has the meaning of Claim 1

and the naphtholactam rings can be substituted as indicated in Claim 1

are condensed with a compound of the formula

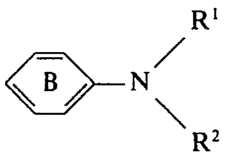

or

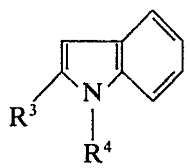

in which

$R^1$, $R^2$, B, $R^3$ and $R^4$ have the meaning indicated in Claim 1 and
the rings can contain the substituents indicated in Claim 1

and with a compound of the formula

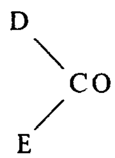

in which

D and E have the meaning indicated in Claim 1 in any order.

6. Methylenebis-naphtholactam compounds of the general formula

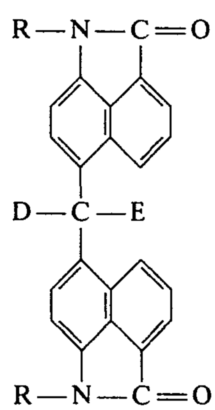

wherein

R, D and E have the meaning indicated in Claim 1 and

the naphtholactam rings can be substituted in the manner indicated in Claim 1.

7. Process for preparing methylenebis-naphtholactam compounds according to Claim 6, characterised in that naphtholactam compounds of the formula

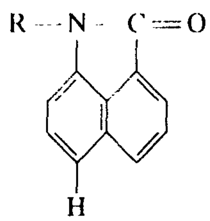

wherein

R has the meaning of Claim 1 and

the naphtholactam ring can be substituted as indicated in Claim 1

are condensed with compounds of the formula

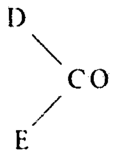

wherein

E and D have the meaning of Claim 1, in particular with formaldehyde.

8. Process for dyeing cationically dyeable fibres of polymers and copolymers of acrylonitrile and of dicyanoethylene and acid-modified fibres of polyamides and polyesters, characterised in that dyestuffs according to Claims 1 to 4 are used.

9. Process for colouring paper, characterised in that dyestuffs according to Claims 1 to 4 are used.

10. Process for spin-dyeing polyacrylonitrile and ist copolmers, characterised in that dyestuffs according to Claims 1 to 4 are used.

## Revendications

1. Colorants cationiques de formule générale

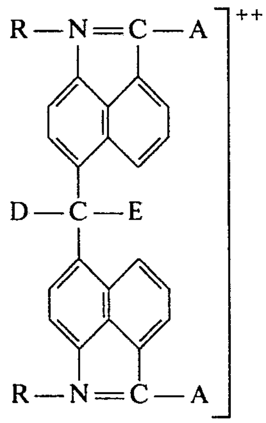

2 An$^{-}$ (I)

et leurs bases de formules respecives

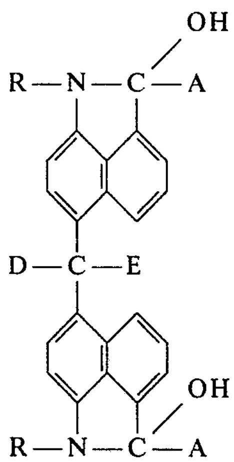

(Ia) et

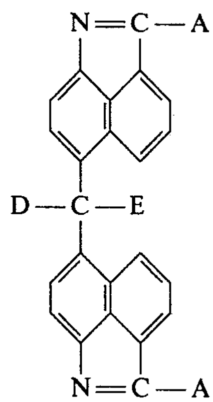

(Ib)

dans lesquelles

R représente l'hydrogène ou un groupe alkyle en $C_1-C_8$ éventuellement substitue par des restes non ioniques,

A représente un reste de formule

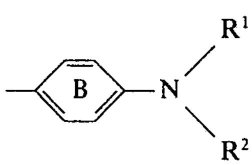

ou

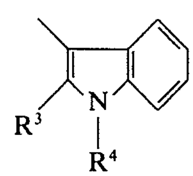

dans laquelle

$R^1$ représente l'hydrogène, un groupe alkyle en $C_1-C_4$ ou cycloalkyle éventuellement substitué par des restes non ioniques et

$R^2$ représente un groupe alkyle en $C_1-C_6$ éventuellement substitué par des restes non ioniques et qui peut le cas échéant fermer un cycle sur le cycle B, ou un groupe aralkyle ou aryle, ou bien

$R^1$ et $R^2$ forment ensemble et avec l'atome d'azote un cycle,

$R^3$ représente un groupe alkyle en $C_1-C_4$, un groupe (alcoxy en $C_1-C_4$) – carbonyle ou un groupe aryle,

$R^4$ représente l'hydrogène ou un groupe alkyle en $C_1-C_4$ éventuellement substitue par des restes non ioniques,

D et E représentant l'hydrogène, un groupe alkyle en $C_1-C_4$ ou alcényle en $C_2-C_4$ éventuellement substitué par des restes non ioniques et D peut en outre représenter un groupe aryle, aralkyle, aralcényle ou un reste hétérocyclique, ou bien D et E forment ensemble et avec l'atome de carbone un cycle, et

An représente un anion,

les cycles pouvant contenir les restes non ioniques usuels dans la chimie des colorants.

2. Colorants cationiques de formule générale

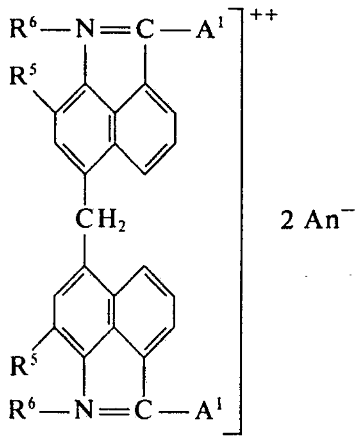

dans laquelle

$R^6$ représente l'hydrogène, un groupe alkyle en $C_1-C_4$ qui peut porter des substituants cyano, hydroxy, alcoxy en $C_1-C_4$, chloro,(alcoxy en $C_1-C_4$)-carbonyle, aminocarbonyle ou mono- ou di-(alkyle en $C_1-C_4$)-aminocarbonyle,

$R^5$ représente l'hydrogène, le chlore ou le brome,

$A^1$ représente le groupe

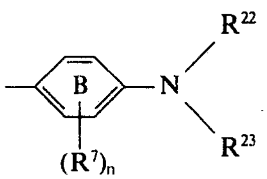

dans lequel

$R^{22}$ représente l'hydrogène ou un groupe alkyle en $C_1-C_6$ éventuellement substitué par un à trois groupes hydroxy, un à trois halogènes, des groupes alcoxy en $C_1-C_4$, cyano, (alkyle en $C_1-C_4$)-carbonyloxy, hydroxyalcoxy en $C_1-C_4$, aminocarbonyle, carboxy, (alcoxy en $C_1-C_4$)-carbonyle ou alcényloxy en $C_3$ ou $C_4$, par des groupes acyloxy, benzyloxy, phényloxy, sulfonamido ou acylamido, un groupe alcényle en $C_2-C_4$ éventuellement substitué par des halogènes ou un groupe cyclohexyle éventuellement substitué par un groupe alkyle en $C_1-C_4$,

$R^{23}$ représente un groupe alkyle en $C_1-C_6$ éventuellement substitué comme $R^{22}$, un groupe benzyle ou phényle, ou forme avec le cycle B une tétrahydroquinoléine, une indoline, un hexahydrocarbazole ou une dihydrobenzoxazine, qui peut porter des groupes alkyle en $C_1-C_4$ et phényle, ou bien

$R^{22}$ et $R^{23}$ forment ensemble un cycle pyrrolidine, pipéridine, morpholine, pyrazoline ou pipérazine qui peut porter des groupes alkyle en $C_1-C_4$ et phényle,

$R^7$ représente un groupe alkyle en $C_1-C_4$, alcoxy en $C_1-C_4$ ou un halogène et

n est un nombre de 1 à 4,

ou bien un groupe

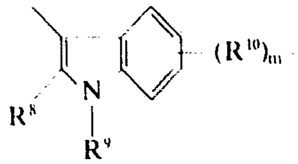

(VI)

dans lequel

$R^8$ représente un groupe alkyle en $C_1-C_4$, un groupe phényle ou un groupe alcoxycarbonyle en $C_1-C_4$,

$R^9$ représente l'hydrogène, un groupe alkyle en $C_1-C_4$ éventuellement substitué par des groupes hydroxy, des halogènes, des groupes alcoxy en $C_1-C_4$, cyano ou acyloxy et

$R^{10}$ représente l'hydrogène, un halogène, un groupe alkyle en $C_1-C_4$, alcoxy en $C_1-C_4$, carbalcoxy contenant 1 à 4 atomes de carbone, alkylsulfonyle en $C_1-C_4$, phénylsulfonyle, acétyle ou benzoyle et

m a une valeur de 1 à 4 et

$An^-$ représente un anion, les restes phényle pouvant être substitués par des groupes alkyle en $C_1-C_4$, alcoxy en $C_1-C_4$, hydroxy, le chlore ou le brome.

3. Colorants selon la revendication 2, dans lesquels

$R^6$ représente l'hydrogène, un groupe alkyle en $C_1-C_4$, $\beta$-cyanéthyle, $\beta$-hydroxyéthyle, $\beta$-alcoxyéthyle, $\beta$-chloréthyle, méthoxycarbonyléthyle, éthoxycarbonyléthyle, méthoxycarbonylméthyle ou éthoxycarbonylméthyle,

$R^5$ représente l'hydrogène et

$A^1$ représente le groupe

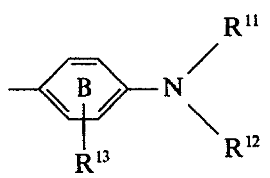

dans lequel

$R^{11}$ représente l'hydrogène ou un groupe alkyle en $C_1-C_4$ éventuellement substitué par des groupes hydroxy, méthoxy, éthoxy, le chlore, des groupes cyano, acétoxy ou acétyle,

$R^{12}$ représente un groupe alkyle en $C_1-C_4$ éventuellement substitué par des groupes hydroxy, méthoxy, éthoxy, le chlore, des groupes cyano, acétoxy ou acétyle, un groupe benzyle ou un groupe phényle éventuellement substitué par le chlore, des groupes méthyle, méthoxy ou éthoxy,

$R^{13}$ représente l'hydrogène, un groupe méthyle, le chlore, un groupe méthoxy ou éthoxy,

un groupe

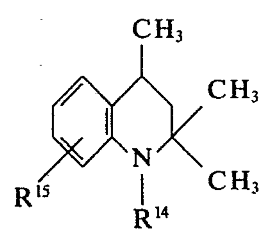

dans lequel

$R^{14}$ représente l'hydrogène ou un groupe alkyle en $C_1-C_4$, hydroxyalkyle en $C_2-C_4$, chloréthyle, brométhyle, méthoxyéthyle, cyanéthyle, acétoxyéthyle, hydroxyéthoxyéthyle, aminocarbonyléthyle, carboxyéthyle, $\gamma$-cyanopropyle, $\beta$-hydroxy-$\gamma$-allyloxy-n-propyle, $\beta$-hydroxy-$\gamma$-méthoxy-n-propyle, $\beta$-hydroxy-$\gamma$-éthoxy-n-propyle, $\beta$-hydroxy-$\gamma$-butoxy-n-propyle, $\beta$-chloro-n-propyle, $\beta$-chloro-n-butyle, $\beta$-chloro-isobutyle, $\beta,\gamma$-dichloro-n-propyle, $\beta$-acétoxy-n-propyle, $\beta$-hydroxy-$\gamma$-chloro-n-propyle, vinyle, allyle, méthallyle, chlorallyle ou cyclohexyle et

$R^{15}$ représente l'hydrogène, un groupe méthoxy ou éthoxy,

ou bien le groupe

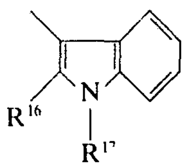

dans lequel

$R^{16}$ représente un groupe méthyle ou un groupe phényle éventuellement substitué par le chlore, des groupes méthyle ou méthoxy et
$R^{17}$ représente un groupe alkyle en $C_1-C_4$ éventuellement substitué par des groupes hydroxy, le chlore, des groupes cyano ou acyloxy, ou l'hydrogène.

4. Colorants de formule générale

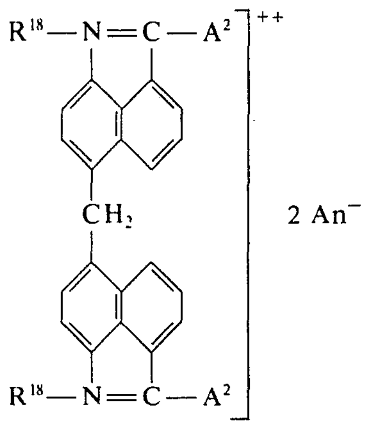

dans laquelle

$R^{18}$ représente un groupe méthyle, éthyle ou $\beta$-cyanéthyle et
$A^2$ représente le groupe

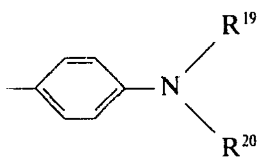

dans lequel

$R^{19}$ représente l'hydrogène, un groupe méthyle, éthyle, cyanéthyle, hydroxyéthyle, acétoxyéthyle, méthoxyéthyle, éthoxyéthyle, chloréthyle, hydroxypropyle, propyle, butyle et
$R^{20}$ représente l'un des groupes alkyle mentionnés pour $R^{19}$ ou un groupe phényle, méthoxy-phényle, éthoxyphényle, méthylphényle ou chlorophényle, ou, en préférence toute particulière,

un groupe

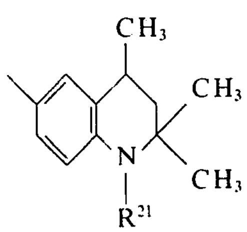

dans lequel

$R^{21}$ représente l'hydrogène, un groupe alkyle en $C_1-C_4$, hydroxyéthyle, hydroxypropyle, cyanéthyle, chloréthyle, acétoxyéthyle, acétoxypropylel, méthoxyéthyle, ethoxyéthyle, hydroxybutyle, hydroxycarbonyléthyle ou hydroxyéthoxyéthyle, et

An représente un anion.

5. Procédé de préparation des colorants selon la revendication 1, caractérisé en ce que l'on condense, dans un ordre quelconque, des dérivés du naphtholactame de formule

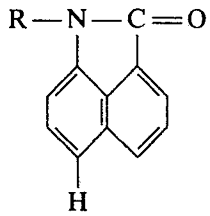

dans laquelle

R a les significations indiquées dans la revendication 1,

et les noyaux du naphtholactame peuvent porter les substituants indiqués dans la revendication 1,

avec un composé de formule

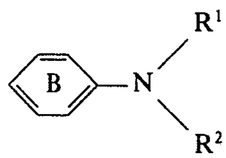

ou

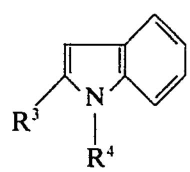

dans laquelle

| | |
|---|---|
| $R^1$, $R^2$, B, $R^3$ et $R^4$ | ont les significations indiquées dans la revendication 1 et |
| les noyaux | peuvent porter les substituants indiqués dans la revendication 1, |

et avec un composé de formule

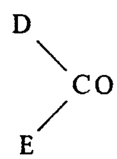

dans laquelle

D et E ont les significations indiquées dans la revendication 1.

6. Dérivés du méthylène-bis-naphtholactame de formule générale

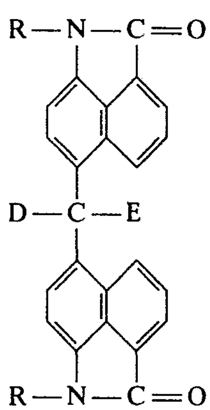

dans laquelle

R, D et E ont les significations indiquées dans la revendication 1 et

les noyaux de naphtholactame peuvent porter les substituants indiqués dans la revendication 1.

7. Procédé de préparation des dérivés du méthylène-bis-naphtholactame selon la revendication 6, caractérisé en ce que l'on condense des dérivés du naphtholactame de formule

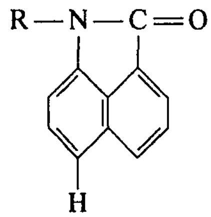

dans laquelle

R a les significations indiquées dans la revendication 1,

et le cycle de naphtholactame peut porter les substituants indiqués dans la revendication 1, avec des composés de formule

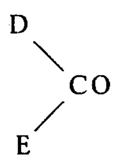

dans laquelle

E et D ont les significations indiquées dans la revendication 1, en particulier le formaldéhyde.

8. Procédé pour teindre des fibres aptes à la teinture par colorants cationiques, en polymères et copolymères de l'acrylonitrile et du dicyanéthylène, ainsi que des fibres de polyamides et de polyesters à modification acide, caractérisé en ce que l'on utilise des colorants selon les revendications 1 à 4.

9. Procédé pour colorer le papier, caractérisé en ce que l'on utilise des colorants selon les revendications 1 à 4.

10. Procédé pour teindre le polyacrylonitrile et ses copolymères au filage, caractérisé en ce que l'on utilise des colorants selon les revendications 1 à 4.